(19) Europäisches Patentamt
European Patent Office
Office européen des brevets

(11) Numéro de publication : **0 023 464**
**B1**

(12) # FASCICULE DE BREVET EUROPÉEN

(45) Date de publication du fascicule du brevet : **07.04.82**

(21) Numéro de dépôt : **80420090.5**

(22) Date de dépôt : **16.07.80**

(51) Int. Cl.[3] : **C 07 F 11/00**, C 07 F 9/02, C 07 C 31/28, C 07 C 29/94

(54) **Procédé de stabilisation des solutions de dérivés organiques du chrome hexavalent dans les solvants hydrocarbonés, par les esters phosphoriques.**

(30) Priorité : **26.07.79 FR 7919738**

(43) Date de publication de la demande : **04.02.81 (Bulletin 81/05)**

(45) Mention de la délivrance du brevet : **07.04.82 Bulletin 82/14**

(84) Etats contractants désignés : **AT BE DE FR GB IT LU NL**

(56) Documents cités :
**US - A - 2 329 707**
**US - A - 3 487 018**

(73) Titulaire : **RHONE-POULENC INDUSTRIES**
**22 Avenue Montaigne**
**F-75008 Paris (FR)**

(72) Inventeur : **De Soyres, Bruno**
**34, rue des Buissons Brunstatt**
**F-68900 Mulhouse (FR)**
Inventeur : **Nouvel, Jacques**
**9, chemin de la Vernique**
**F-69160 Tassin-La-Demi-Lune (FR)**

(74) Mandataire : **Chichery, Guy et al**
**RHONE-POULENC INDUSTRIES Centre de Recherches des Carrières Service BREVETS**
**F-69190 Saint-Fons (FR)**

**EP 0 023 464 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

Procédé de stabilisation des solutions de dérivés organiques du chrome hexavalent dans les solvants hydrocarbonés, par les esters phosphoriques

La présente invention a pour objet un procédé de stabilisation de solutions de dérivés organiques du chrome hexavalent dans les solvants hydrocarbonés.

On sait que les composés organiques du chrome et notamment ses sels d'acide carboxylique, ses chélates avec les composés dicarbonylés ou les chromates d'alcools tertiaires sont utilisés comme catalyseurs de diverses réactions telles que la dimérisation de composés dioléfiniques, l'oxydation d'hydrocarbures cycloaliphatiques ou alcoylaromatiques par l'air, l'hydrogénation de doubles liaisons oléfiniques de polymères hydrocarbonés, la décomposition d'hydroperoxydes organiques ou la réticulation de polymères à groupes carboxyliques par des composés à groupes époxy. Les chromates d'alcools tertiaires sont encore utilisés comme oxydants.

Pour la réalisation de ces diverses réactions il peut être avantageux de mettre en œuvre les dérivés du chrome sous forme d'une solution dans un solvant hydrocarboné tel que les alcanes, les cycloalcanes ou les hydrocarbures aromatiques. Il est souvent commode, dans le cas de réactions conduites en continu, de disposer d'une réserve de solution catalytique. On a cependant constaté que ces solutions ne sont pas stables dans le temps et donnent lieu à la précipitation du métal sous forme d'oxyde de chrome. Un tel phénomène est particulièrement gênant car il provoque à la fois des bouchages des pompes et conduites d'alimentation et des variations de concentrations des solutions catalytiques, ce qui peut être préjudiciable au déroulement régulier des réactions concernées.

Ce phénomène de précipitation est d'autant plus accentué que les solutions sont stockées plus longtemps. La présente invention a précisément pour objet de résoudre ce problème de précipitation du chrome à partir des solutions organiques de ses dérivés au cours du temps.

Plus spécifiquement, la présente invention concerne un procédé de stabilisation de solutions dans des solvants hydrocarbonés de composés du chrome hexavalent, caractérisé en ce que l'on ajoute aux dites solutions un ester phosphorique comportant au moins un groupement acide libre et soluble dans le solvant hydrocarboné.

On peut mettre en œuvre un ester o.phosphorique ou un ester d'acide polyphosphorique ou bien encore un mélange de ces divers esters. Les esters phosphoriques ou polyphosphoriques sont des composés qui sont bien décrits dans la littérature et en particulier dans le traité de HOUBEN-WEYL : Methoden der organischen chemie band XII/2 ; Organische phosphorverbindungen (1964).

Parmi les esters orthophosphoriques utilisables, on peut citer ceux de formule générale :

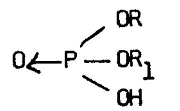

(I)

dans laquelle R représente un radical hydrocarboné saturé, comportant de 1 à 18 atomes de carbone et $R_1$ représente un atome d'hydrogène ou l'un des radicaux que représente R. Les radicaux R et $R_1$ peuvent être identiques ou différents. Parmi les esters de formule (I) on préfère utiliser ceux dans lesquels l'un au moins des radicaux R ou $R_1$ possède plus de 3 atomes de carbone.

Le symbole R peut représenter par exemple un radical alcoyle linéaire ou ramifié tel que : méthyle, éthyle, isopropyle, butyle, t-butyle, heptyle, octyle, dodécyle, octadécyle ; un radical cycloalcoyle tel que les radicaux cyclohexyle, cyclooctyle, cyclododécyle, mentyle ; un radical aryle tel que phényle, tolyle ; un radical aralcoyle tel que les radicaux benzyle et phényléthyle.

Parmi les esters orthophosphoriques auxquels on peut faire appel figurent notamment l'orthophosphate de méthyle et de n-butyle ; d'éthyle et d'octyle ; de monobutyle ; de dibutyle ; de disobutyle ; de monoheptyle ; de diheptyle ; de mono(éthyl-2 hexyle) ; de di(éthyl-2 hexyle) ; de monodécyle ; de didodécyle ; de monooctadécyle ; de dioctadécyle ; de méthyle et de cyclohexyle ; de monocyclohexyle ; de dicyclohexyle ; l'orthophosphate de monobenzyle ; l'orthophosphate de méthyle et de benzyle ; l'orthophosphate d'éthyle et de tolyle ; l'orthophosphate de monomentyle ; l'orthophosphate de n-dodécyle et de cyclohexyle.

Dans les phosphates de formule (I) la nature du reste R ne présente pas de caractère critique pour la solution du problème envisagé ; il suffit qu'elle assure la solubilité du phosphate dans les solvants hydrocarbonés. Préférentiellement, R est un radical alcoyle ramifié.

Parmi les esters polyphosphoriques on peut citer les esters des acides pyrophosphoriques, les esters de tripolyphosphates... On mentionnera tout particulièrement les esters des acides pyrophosphoriques, ceux-ci pouvant être représentés par la formule suivante :

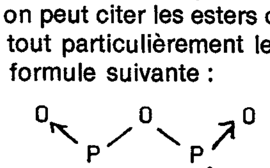

(II)

les divers symboles $R_1$ et R ayant la signification préalablement donnée. A titre illustratif, on citera les

esters pyrophosphoriques acides suivants : le pyrophosphate de diméthyle, le pyrophosphate de diéthyle, le pyrophosphate de dibutyle, le pyrophosphate de diisobutyle, le pyrophosphate de dioctyle, le pyrophosphate de di-n éthyl-2 hexyle, le pyrophosphate de butyle et de benzyle...

Pour mettre en œuvre le procédé selon l'invention, on peut utiliser un seul ester ou bien un mélange de deux ou de plus de deux esters phosphoriques. La nature des divers esters n'est pas critique : elle doit, comme on l'a déjà dit, assurer simplement la solubilité des phosphates dans les solvants hydrocarbonés. Le mélange peut être à base d'o.phosphates, ou de pyrophosphates, ou encore être constitué par au moins un o.phosphate et un pyrophosphate. Selon la nature de la solution de dérivé organique du chrome, on peut avoir intérêt à utiliser un mélange d'esters constitué par un o.phosphate et un pyrophosphate. En règle générale, et dans le cas des solutions de chromates d'alkyle tertiaire, un tel mélange renferme avantageusement et en poids de 5 à 30 % du dérivé « pyro ».

Ces mélanges peuvent être des mélanges synthétiques ou être directement obtenus par exemple par action des alcanols sur $P_2O_5$ ou sur un mélange d'acide o.phosphorique et polyphosphorique.

Les mélanges d'esters phosphoriques contiennent des fonctions acides libres et peuvent être constitués par des esters renfermant, et rapporté à l'atome de phosphore, un nombre identique ou différent de groupements acides libres. Cette formule est avantageuse : on peut ainsi facilement déterminer et choisir la teneur en groupement acide libre qui corresponde à la meilleure stabilité et à la meilleure solubilité. Dans un tel contexte, on peut citer les mélanges renfermant de 50 à 90 % d'o.phosphate de dialkyle, le restant étant par exemple constitué par un pyrophosphate de dialkyle, ou par un o.phosphate de monoalkyle.

On a mis en évidence qu'un tel mélange convenait particulièrement bien pour stabiliser les solutions dans les solvants hydrocarbonés de chromates d'alkyle tertiaire.

Les dérivés du chrome hexavalent, en solution dans les solvants hydrocarbonés et qui sont stabilisés par les dérivés phosphoriques précédemment définis peuvent être de nature variée. Ces dérivés sont en général obtenus à partir de l'anhydride chromique $CrO_3$ et leur constitution chimique correspond plus particulièrement à celles des anions $[Cr_nO_{3n+1}]^{2-}$ n étant un nombre entier égal à 1, 2, 3 ou 4. Ces dérivés sont par exemple décrits dans les ouvrages suivants :

— Gmelin Handbuch der anorganischen Chemie ; Chrom teil C (1965).

— Pascal, traité de Chimie minérale, tome XIV.

Le procédé selon l'invention s'applique tout particulièrement bien à la stabilisation des chromates d'alcools tertiaires aliphatiques et cycloaliphatiques comportant de 4 à 20 atomes de carbone, tels que l'alcool tertiobutylique, l'alcool t-amylique, le méthyl-2 pentanol-3, le méthyl-2 hexanol-2, le diméthylpentadécyl-carbinol, le méthyl-2 cyclohexanol, l'éthyl-1 cyclohexanol. Ces composés sont obtenus en faisant réagir l'anhydride chromique avec les alcools tertiaires selon les techniques par exemple décrites dans le brevet américain 3.287.082 et dans Journal of American Chemical Society *78*, 1694-8 (1955).

Comme exemples particuliers de chromates d'alcools tertiaires on peut citer le chromate de t-butyle ; le chromate de t-amyle ; le chromate de diméthylpentadécylcarbinol ; le chromate de méthyl-1 cyclohexyle.

Comme exemples de solvants hydrocarbonés dont les solutions de dérivés du chrome peuvent être stabilisées par adjonction de phosphates acides solubles, on peut citer : les alcanes linéaires ou ramifiés comportant de 5 à 20 atomes de carbone comme le n-pentane ; le n-hexane ; le méthyl-2 pentane ; le méthyl-3 pentane ; le diméthyl-2,3 butane ; le n-heptane ; le n-pentadécane ; les cycloalcanes comportant de 5 à 9 atomes de carbone et comportant éventuellement de 1 à 3 groupes alcoyles ayant de 1 à 4 atomes de carbone comme le cyclopentane ; le cyclohexane ; le cycloheptane ; le cyclooctane ; le méthylcyclohexane ; des hydrocarbures aromatiques comme le benzène ; le toluène ; les xylènes ; l'éthylbenzène. Le procédé selon l'invention s'applique tout particulièrement bien à la stabilisation de solution de dérivés du chrome (VI) dans les cycloalcanes.

La concentration des solutions hydrocarbonées de dérivés solubles du chrome n'est pas critique et peut varier dans de larges limites, par exemple entre des valeurs allant de 0,1 g/l de composé à la limite de solubilité du dérivé du chrome dans le solvant considéré à la température de 20 °C.

La quantité de phosphate acide soluble ajouté à la solution de composé du chrome peut varier également dans de larges limites en fonction du dérivé du chrome considéré et du phosphate utilisé. En général une quantité de phosphate introduisant entre 0,001 et 2,5 atomes-gramme de phosphore par atome-gramme de chrome convient bien, quoique l'on puisse sortir sans inconvénient de ces limites. De préférence il suffit de mettre en œuvre des quantités de phosphate introduisant de 0,01 à 1 atome-gramme de phosphore par atome-gramme de chrome.

Pour mettre en pratique le procédé de l'invention il suffit d'ajouter le phosphate-acide ou le mélange de dérivés phosphoriques acides à la solution de dérivé du chrome maintenue à température ambiante ou portée à température plus élevée pour faciliter la dissolution du stabilisant. On peut également ajouter le dérivé du chrome à la solution organique du stabilisant ou bien dissoudre simultanément le dérivé du chrome et le phosphate-acide dans le solvant choisi.

Les exemples suivants illustrent l'invention et montrent comment elle peut être mise en pratique.

## Exemple 1

On prépare une solution cyclohexanique de chromate de t-butyle en faisant réagir 190 g d'une

solution aqueuse de $CrO_3$ à 30,2 % de chrome métal avec 440 $cm^3$ de t-butanol dans 1 540 $cm^3$ de cyclohexane en présence de 130 $cm^3$ d'eau. On opère à 20 °C. Le mélange réactionnel est maintenu 1 heure sous agitation puis la couche aqueuse est éliminée par décantation. On obtient de cette façon une solution cyclohexanique de chromate de t-butyle contenant 2,95 % en poids de chrome métallique.

Lorsqu'on conserve cette solution à température ambiante, on constate l'apparition d'un précipité de couleur brun-rouge après 2 heures de stockage.

Si l'on ajoute 1 g d'orthophosphate de monooctyle à 150 g de solution cyclohexanique (rapport du nombre d'at.g de phosphore au nombre d'at.g de Cr = 0,056) on ne note la formation d'aucun précipité après 240 heures de stockage.

## Exemple 2

Dans 150 g de la solution cyclohexanique de l'exemple 1 on ajoute 1 g d'orthophosphate de monoisobutyle : rapport P/Cr = 0,076. On ne note aucun dépôt après 240 heures de stockage.

## Exemple 3

Selon le mode opératoire de l'exemple 1, on prépare une solution cyclohexanique de chromate de tertiobutyle à 10 %.

Dans une série de tubes à fond plat on introduit 150 g de la solution cyclohexanique de chromate de tertio-butyle et 0,5 g d'ester phosphorique ou de mélange d'esters phosphoriques. Après une brève agitation, les tubes sont abandonnés à 25° durant 300 h.

On observe alors que le tube témoin sans stabilisant renferme 1,3 g de précipité alors que les quantités de précipité sont bien inférieures dans les divers tubes ayant reçu des stabilisants.

Les résultats d'essais figurent dans le tableau qui suit

| | Stabilisant phosphorique | | | Résultats de stabilisation (Masse de précipité après 300 h) |
|---|---|---|---|---|
| | Ester o.phosphorique | Ester pyrophosphorique | % pondéral ester o.phosphorique / totalité ester phosphorique | |
| Essai n° 1 | isobutyle | isobutyle | 97 | 0,6 gramme |
| 2 | isobutyle | isobutyle | 92 | 0,05 gramme |
| 3 | isobutyle | isobutyle | 83 | 0 |
| 4 | isobutyle | isobutyle | 70 | 0,1 gramme |
| 5 | éthyl-2 hexyle | éthyl-2 hexyle | 92 | 0,12 gramme |

**Revendications**

1. Procédé de stabilisation de solutions dans les solvants hydrocarbonés de composés organiques du chrome hexavalent, caractérisé en ce que l'on ajoute aux dites solutions un ester phosphorique comportant au moins un groupement acide libre et soluble dans le solvant hydrocarboné.

2. Procédé selon la revendication 1 dans lequel le composé organique du chrome hexavalent est un chromate d'alcool tertiaire.

3. Procédé selon l'une des revendications précédentes dans lequel l'ester phosphorique est un o.phosphate répondant à la formule générale :

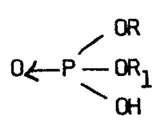 (I)

dans laquelle

R représente un radical hydrocarboné saturé comportant de 1 à 18 atomes de carbone ;

$R_1$ représente un atome d'hydrogène ou l'un des radicaux que représente R.

4. Procédé selon la revendication 3 dans lequel on utilise un ester o.phosphorique de formule I dans laquelle l'un au moins des radicaux R et $R_1$ comporte au moins 3 atomes de carbone.

5. Procédé selon l'une des revendications 1 et 2 dans lequel l'ester phosphorique est un pyrophosphate répondant à la formule générale II :

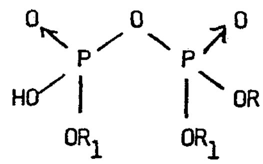

(II)

dans laquelle

R représente un radical hydrocarboné saturé comportant de 1 à 18 atomes de carbone ;

$R_1$ représente un atome d'hydrogène ou l'un des radicaux que représente R.

6. Procédé selon l'une des revendications 1 et 2 dans lequel on utilise en tant que stabilisant un mélange d'ester o.phosphorique de formule I :

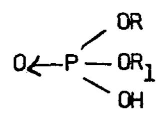

(I)

et d'ester pyrophosphorique de formule II :

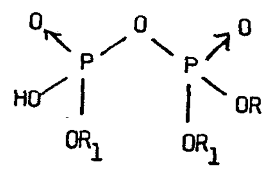

(II)

le pourcentage exprimé pondéralement, en ester o.phosphorique étant compris entre 70 et 95 %, et les symboles R et $R_1$ ayant la signification suivante :

R représente un radical hydrocarboné saturé comportant de 1 à 18 atomes de carbone ;

$R_1$ représente un atome d'hydrogène ou l'un des radicaux que représente R.

7. Procédé selon l'une des revendications 1 à 6, caractérisé en ce que la quantité d'ester phosphorique ou de mélange d'ester phosphorique utilisé est introduit dans la solution de composé du chrome à raison de 0,001 à 2,5 at.g de phosphore par at.g de chrome en solution.

8. Procédé selon l'une des revendications 1 à 4 caractérisé en ce que la solution est une solution d'un chromate d'alcool tertiaire dans un cycloalcane.

9. Procédé selon la revendication 5 caractérisé en ce que la solution est une solution d'un chromate d'alcool tertiaire dans le cyclohexane.

**Claims**

1. Process for the stabilisation of solutions of organic compounds of hexavalent chromium in hydrocarbon solvents, characterised in that a phosphoric acid ester which contains at least one free acid group and is soluble in the hydrocarbon solvent is added to the said solutions.

2. Process according to claim 1, in which the organic compound of hexavalent chromium is the chromate of a tertiary alcohol.

3. Process according to one of the preceding claims, in which the phosphoric acid ester is an orthophosphate corresponding to the general formula :

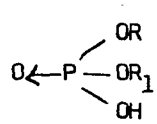

(I)

in which

R represents a saturated hydrocarbon radical containing from 1 to 18 carbon atoms and

$R_1$ represents a hydrogen atom or one of the radicals represented by R.

4. Process according to claim 3, in which an orthophosphoric acid ester of the formula I in which at least one of the radicals R and $R_1$ contains at least 3 carbon atoms is used.

5. Process according to one of claims 1 and 2, in which the phosphoric acid ester is a pyrophosphate

corresponding to the general formula II :

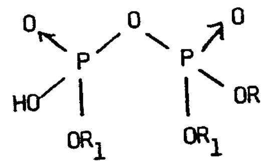

(II)

in which

R represents a saturated hydrocarbon radical containing from 1 to 18 carbon atoms and

$R_1$ represents a hydrogen atom or one of the radicals represented by R.

6. Process according to one of claims 1 and 2, in which a mixture of an orthophosphoric acid ester of the formula I :

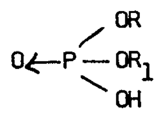

(I)

and a pyrophosphoric acid ester of the formula II :

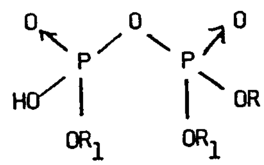

(II)

is used as the stabiliser, the percentage of the orthophosphoric acid ester being between 70 and 95 %, expressed by weight, and the symbols R and $R_1$ having the following meaning :

R represents a saturated hydrocarbon radical containing from 1 to 18 carbon atoms and

$R_1$ represents a hydrogen atom or one of the radicals represented by R.

7. Process according to one of claims 1 to 6, characterised in that the amount of phosphoric acid ester or mixture of phosphoric acid esters used is introduced into the solution of chromium compound at a rate of 0,001 to 2,5 g atoms of phosphorus per g. atom of chromium in solution.

8. Process according to one of claims 1 to 4, characterised in that the solution is a solution of the chromate of a tertiary alcohol in a cycloalkane.

9. Process according to claim 5, characterised in that the solution is a solution of the chromate of a tertiary alcohol in cyclohexane.

**Ansprüche**

1. Verfahren zur Stabilisierung von Lösungen von hexavalenten organischen Chromverbindungen in Kohlenwasserstofflösungsmitteln, dadurch gekennzeichnet, daß man den Lösungen einen im Kohlenwasserstofflösungsmittel löslichen Phosphorsäureester mit mindestens einer freien Säuregruppe zusetzt.

2. Verfahren nach Anspruch 1, dadurch gekennzeichnet, daß die hexavalente organische Chromverbindung ein Chromat eines tertiären Alkohols ist.

3. Verfahren nach einem vorhergehenden Ansprüche, dadurch gekennzeichnet, daß der Phosphorsäureester ein o-Phosphat der allgemeinen Formel :

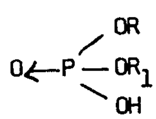

(I)

ist, worin

R einen gesättigten Kohlenwasserstoffrest mit 1 bis 18 Kohlenstoffatomen darstellt,

$R_1$ ein Wasserstoffatom oder einen der für R angegebenen Reste darstellt.

4. Verfahren nach Anspruch 3, dadurch gekennzeichnet, daß man einen o-Phosphorsäureester der Formel I verwendet, worin mindestens einer der Reste R und $R_1$ mindestens 3 Kohlenstoffatome aufweist.

5. Verfahren nach einem der Ansprüche 1 und 2, dadurch gekennzeichnet, daß der Phosphorsäureester ein Pyrophosphat der allgemeinen Formel II

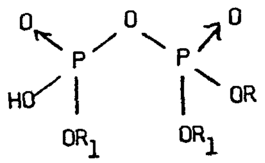

(II)

ist, worin

R einen gesättigten Kohlenwasserstoffrest mit 1 bis 18 Kohlenstoffatomen darstellt,

$R_1$ ein Wasserstoffatom oder einen der für R angegebenen Reste darstellt.

6. Verfahren nach einem der Ansprüche 1 und 2, dadurch gekennzeichnet, daß man als Stabilisator eine Mischung von o-Phosphorsäureester der Formel I:

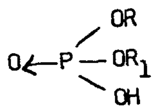

(I)

und Pyrophosphorsäureester der Formel II

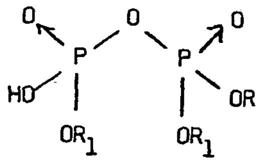

(II)

verwendet, wobei der Gewichtsanteil an o-Phosphorsäureester zwischen 70 und 95 % beträgt und die Symbole R und $R_1$ folgende Bedeutung haben:

R stellt einen gesättigten Kohlenwasserstoffrest mit 1 bis 18 Kohlenstoffatomen dar

$R_1$ stellt ein Wasserstoffatom oder einen der für R angegebenen Reste dar.

7. Verfahren nach einem der Ansprüche 1 bis 6, dadurch gekennzeichnet, daß der Phosphorsäureester oder die Phosphorsäureestermischung in die Lösung der Chromverbindung in einer Menge von 0,001 bis 2,5 Grammatom Phosphor pro Grammatom Chrom in der Lösung eingeführt wird.

8. Verfahren nach einem der Ansprüche 1 bis 4, dadurch gekennzeichnet, daß die Lösung eine Lösung von einem Chromat eines tertiären Alkohols in einem cycloalkan ist.

9. Verfahren nach Anspruch 5, dadurch gekennzeichnet, daß die Lösung eine Lösung von einem Chromat eines tertiären Alkohols in Cyclohexan ist.